# EUROPEAN PATENT APPLICATION

(11) **EP 4 596 526 A1**
(43) Date of publication of application: **06.08.2025**
(21) Application number: 23872350.6
(22) Date of filing: 26.09.2023
(51) Int. Cl.: C07C 17/25, C07B 61/00, C07C 17/358, C07C 21/18

(54) **METHOD FOR PRODUCING DIFLUOROETHYLENE**

(30) Priority: 28.09.2022 JP 2022155045
(71) Applicant: DAIKIN INDUSTRIES, LTD., Osaka-shi, Osaka 530-0001 (JP)
(72) Inventor: CHAKI, Takehiro, Osaka-Shi, Osaka 530-0001 (JP); HASUMOTO, Yuuta, Osaka-Shi, Osaka 530-0001 (JP); NOGUCHI, Atsushi, Osaka-Shi, Osaka 530-0001 (JP); TOGO, Norihiro, Osaka-Shi, Osaka 530-0001 (JP)
(74) Representative: Hoffmann Eitle
(86) International application number: PCT/JP2023/034964
(87) International publication number: WO 2024/071127

(57) **Abstract**

The present disclosure provides a method for more efficiently obtaining HFO-1132(E) and/or HFO-1132(Z) than in conventional methods.

The present disclosure specifically provides a method for producing a composition comprising trans-1,2-difluoroethylene (HFO-1132(E)) and/or cis-1,2-difluoroethylene (HFO-1132(Z)), the method comprising:
(x) supplying a starting material gas comprising 1,1,2-trifluoroethane (HFC-143), HFO-1132(E) and/or HFO-1132(Z), and a diluent gas to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein the content of the diluent gas in the starting material gas is 1 to 80 mol%.

## Description

### Technical Field

The present disclosure relates to a method for producing difluoroethylene.

### Background Art

Non-patent Literature (NPL) 1 discloses a method for isomerizing HFO-1132(Z) into HFO-1132(E) by bringing iodine (catalyst) into contact with HFO-1132(Z) in a gas phase. Patent Literature (PTL) 1 discloses a method for producing a composition comprising HFO-1132(E) and/or HFO-1132(Z), the method comprising steps (x) to (z):
(x) supplying HFC-143 and a composition comprising HFO-1132(E) and/or HFO-1132(Z) to a reactor filled with a catalyst to perform a dehydrofluorination reaction of HFC-143 and an isomerization reaction of HFO-1132(E) and HFO-1132(Z), thereby obtaining a composition comprising HFO-1132(E) and HFO-1132(Z);
(y) separating the reaction product obtained in step (x) into a first stream comprising HFO-1132(E) as a main component, and a second stream comprising HFO-1132(Z) as a main component; and
(z) recycling the first stream or the second stream obtained in step (y) to step (x), to subject the first stream or the second stream to the isomerization reaction.

### Citation List

### Non-patent Literature

NPL 1: Journal of the American Chemical Society, 1961, vol. 83, 3047

### Patent Literature

PTL 1: JP2019-214535A

### Summary of Invention

### Technical Problem

An object is to provide a method for efficiently obtaining HFO-1132(E) and/or HFO-1132(Z).

### Solution to Problem

### Item 1.

A method for producing a composition comprising trans-1,2-difluoroethylene (HFO-1132(E)) and/or cis-1,2-difluoroethylene (HFO-1132(Z)), the method comprising:
(x) supplying a starting material gas comprising 1,1,2-trifluoroethane (HFC-143), HFO-1132(E) and/or HFO-1132(Z), and a diluent gas to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein the content of the diluent gas in the starting material gas is 1 to 80 mol%.

### Item 2.

A method for producing a composition comprising trans-1,2-difluoroethylene (HFO-1132(E)) and/or cis-1,2-difluoroethylene (HFO-1132(Z)), the method comprising:
(x) supplying a starting material gas comprising 1,1,2-trifluoroethane (HFC-143), and HFO-1132(E) and/or HFO-1132(Z) to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein a contact time represented by the ratio of the amount of the catalyst contained W (g) to the flow rate of the starting material gas Fo (flow rate at 0°C and 1 atmosphere: cc/sec), W/Fo, is 0.1 to 10 g·sec/cc.

### Item 3.

The production method according to Item 1, wherein the diluent gas is at least one member selected from the group consisting of HF gas, O₂ gas, CO₂ gas, and N₂ gas.

### Item 4.

The production method according to Item 1 or 2, further comprising:
(y) separating the reaction product obtained in step (x) into a first stream comprising HFO-1132(E) as a main component and a second stream comprising HFO-1132(Z) as a main component.

### Item 5.

The production method according to Item 4, further comprising:
(z) recycling the first stream or the second stream obtained in step (y) to step (x) to subject the first stream or the second stream to the isomerization reaction.

### Advantageous Effects of Invention

The present disclosure enables HFO-1132(E) and/or HFO-1132(Z) to be more efficiently obtained than in conventional methods.

### Brief Description of Drawings

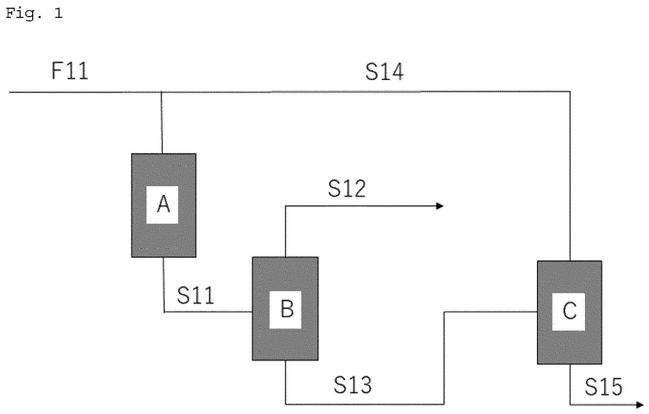

Fig. 1 schematically shows the production process of HFO-1132(E) in Examples 1 to 12.

### Description of Embodiments

An object of the present disclosure is to provide a method for obtaining trans-1,2-difluoroethylene (HFO-1132(E)) and/or cis-1,2-difluoroethylene (HFO-1132(Z)) more efficiently than in conventional methods, in a method for producing a composition comprising HFO-1132(E) and/or HFO-1132(Z), the method comprising supplying a starting material gas comprising 1,1,2-trifluoroethane (HFC-143), and HFO-1132(E) and/or HFO-1132(Z) to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z).

The present inventors conducted extensive research to achieve the above object and found that the object can be achieved by a method using a starting material gas comprising a specific amount of a diluent gas, or a method in which the contact time represented by the ratio of the amount of a catalyst contained W (g) to the flow rate of a starting material gas Fo (flow rate at 0°C and 1 atmosphere: cc/sec), W/Fo, is set to a specific range. The present disclosure has been completed as a result of further research based on this finding. The present disclosure includes the following embodiments.

The method for producing a composition comprising HFO-1132(E) and/or HFO-1132(Z) according to the present disclosure can be roughly classified into the following two embodiments.

### Embodiment 1: Embodiment Using Diluent Gas

A method for producing a composition comprising trans-1,2-difluoroethylene (HFO-1132(E)) and/or cis-1,2-difluoroethylene (HFO-1132(Z)), the method comprising
(x) supplying a starting material gas comprising 1,1,2-trifluoroethane (HFC-143), HFO-1132(E) and/or HFO-1132(Z), and a diluent gas to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein the content of the diluent gas in the starting material gas is 1 to 80 mol%.

### Embodiment 2: Embodiment in which W/Fo Ratio is Specific W/Fo Ratio

A method for producing a composition comprising trans-1,2-difluoroethylene (HFO-1132(E)) and/or cis-1,2-difluoroethylene (HFO-1132(Z)), the method comprising
(x) supplying a starting material gas comprising 1,1,2-trifluoroethane (HFC-143), and HFO-1132(E) and/or HFO-1132(Z) to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein a contact time represented by the ratio of the amount of the catalyst contained W (g) to the flow rate of the starting material gas Fo (flow rate at 0°C and 1 atmosphere: cc/sec), W/Fo, is 0.1 to 10 g·sec/cc.

Terms common to Embodiments 1 and 2 are described first, and then Embodiments 1 and 2 are described below.

### 1. Explanation of Terms

### 1.1. Isomerization Reaction

The isomerization reaction as used herein is an isomerization reaction between HFO-1132(E) and HFO-1132(Z). This isomerization reaction follows the reaction scheme described below. Because HFO-1132(E) is thermodynamically less stable than HFO-1132(Z), this equilibrium is biased to HFO-1132(Z). When a composition comprising HFO-1132(E) and/or HFO-1132(Z) is subjected to an isomerization reaction, the reaction proceeds so as to approach the content ratio of HFO-1132(E) and HFO-1132(Z) at equilibrium. This content ratio is dependent on temperature. By performing the reaction at a higher temperature, the content of HFO-1132(Z) is reduced, and the content of HFO-1132(E) is increased.

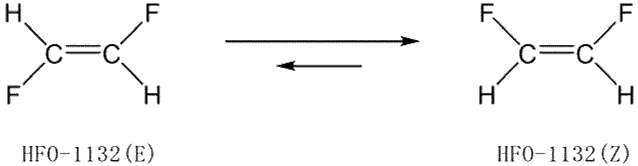

### 1.2. HFO-1132(E) and/or HFO-1132(Z), and Composition Comprising HFO-1132(E) and/or HFO-1132(Z)

HFO-1132(E) and/or HFO-1132(Z) used as a starting material for isomerization may contain other components. The other components can be any component that does not greatly interfere with the isomerization reaction.

Examples of such other components include impurities incorporated in the process of obtaining the composition comprising HFO-1132(E) and/or HFO-1132(Z), and generated by-products. The incorporated impurities include impurities etc. contained in the starting material for obtaining the composition.

Examples of the method for obtaining a composition comprising HFO-1132(E) and/or HFO-1132(Z), which is used as a starting material for isomerization, include a method of subjecting halogenated ethane to a dehydrohalogenation reaction or a dehalogenation reaction to obtain the composition; and the like.

The halogenated ethane used in the reaction is not limited, and can be selected from a wide range. Specific examples include the halogenated ethane described below. Such halogenated ethane is used in a wide variety of applications, such as refrigerants, solvents, blowing agents, and propellants; and is generally commercially available.

1,1,2-Trifluoroethane (CHF₂CH₂F; HFC-143)
1-Bromo-1,2-difluoroethane (CHFBrCH₂F)
1-Chloro-1,2-difluoroethane (CHClFCH₂F)
1,2-Dichloro-1,2-difluoroethane (CHClFCHClF)
1,1,2,2-Tetrafluoroethane (CHF₂CHF₂)
1-Chloro-1,2,2-trifluoroethane (CHClFCHF₂)

### 1.3. Dehydrohalogenation Reaction of Halogenated Ethane

The dehydrohalogenation reaction of halogenated ethane is preferably performed in the presence of a catalyst. The catalyst is not limited and can be selected from a wide range.

As the catalyst, known catalysts that can be used for a dehydrohalogenation reaction can be used. Examples include halides, oxides, and oxidized halides of transition metals, elements that belong to group 14 and group 15, and Mg, Al, etc. Specific examples of transition elements include Ti, V, Cr, Mn, Fe, Co, Ni, Cu, Zn, Zr, Nb, Ta, and W. Specific examples of elements that belong to group 14 include Sn and Pb. Specific examples of elements that belong to group 15 include Sb and Bi.

Examples of halides of these elements include fluorides and chlorides.

Of these, examples of preferable catalysts include SbCl₅, SbCl₃, SbF₅, TaCl₅, SnCl₄, NbCl₅, FeCl₃, CrCl₃, CrF₃, TiCl₄, MoCl₅, Cr₂O₃, CrO₂, CrO₃, CoCl₂, NiCl₂, MgF₂, AlOF (fluorinated aluminum oxide), and CrOF (fluorinated chromium oxide).

These catalysts may be used singly, or in a combination of two or more. These catalysts may be supported on a carrier. Examples of carriers include, but are not limited to, porous alumina silicate typified by zeolite, aluminum oxide, silicon oxide, activated carbon, titanium oxide, zirconia oxide, zinc oxide, and aluminum fluoride. These may be used singly or in a combination of two or more, or a structural composite form thereof may be used. Specific examples of catalysts supported on a carrier include Cr₂O₃/Al₂O₃, Cr_{y}O₃/AlF₃, Cr₂O₃/C, CoCl₂/Cr₂O₃/Al₂O₃, NiCl₂/Cr₂O₃/Al₂O₃, CoCl₂/AlF₃, and NiCl₂/AlF₃.

As the catalyst, a chromium-containing catalyst is preferably used, and chromium oxide is particularly preferably used. Examples of chromium oxide include crystalline chromium oxide, amorphous chromium oxide, and the like, with the chromium oxide subjected to fluorination being particularly preferable.

Specific examples of the method for obtaining a composition comprising HFO-1132(E) and/or HFO-1132(Z) by subjecting halogenated ethane to a dehydrohalogenation reaction include a method comprising bringing HFC-143 used as a starting material into contact with fluorinated chromium oxide used as a catalyst at 400°C at a contact time W/Fo (cat./ml·s) = 60 sec, under a 15 mol% O₂ atmosphere.

### 1.4. Dehalogenation Reaction of Halogenated Ethane

The dehalogenation reaction of halogenated ethane can be performed according to a known method. For example, the dehalogenation reaction can be performed by a reaction with a metal reagent, such as an organomagnesium compound or zinc, in an aprotic solvent. As a specific example, 1-bromo-1,2,2-fluoroethane can be reacted with a zinc powder in an aprotic solvent to perform deFBr(defluorination and debromination), thereby obtaining a composition comprising HFO-1132(E) and/or HFO-1132(Z).

The reaction temperature of the dehalogenation reaction is not limited, and can be suitably set. For example, the reaction temperature can be set to -20 to 200°C, and preferably 0 to 80°C.

The metal reagent used in the dehalogenation reaction is not limited and can be suitably determined. Usable examples include zinc, magnesium, and nickel. Of these, zinc is preferable.

### 2. Embodiment 1: Embodiment Using Diluent Gas

The method for producing difluoroethylene according to Embodiment 1 is a method for producing a composition comprising trans-1,2-difluoroethylene (HFO-1132(E)) and/or cis-1,2-difluoroethylene (HFO-1132(Z)), the method comprising
(x) supplying a starting material gas comprising 1,1,2-trifluoroethane (HFC-143), HFO-1132(E) and/or HFO-1132(Z), and a diluent gas to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein the content of the diluent gas in the starting material gas is 1 to 80 mol%.

In step (x) in the method according to Embodiment 1, a starting material gas comprising HFC-143, HFO-1132(E) and/or HFO-1132(Z), and a diluent gas is supplied to a reactor containing a catalyst to perform a dehydrofluorination reaction of HFC-143 and an isomerization reaction between HFO-1132(E) and HFO-1132(Z), thereby obtaining a composition comprising HFO-1132(E) and HFO-1132(Z). In the method according to Embodiment 1, since the content of the diluent gas in the starting material gas is 1 to 80 mol%, the composition can be more easily separated into a first stream comprising HFO-1132(E) as a main component and a second stream comprising HFO-1132(Z) as a main component in the subsequent step (y). This allows the production method of the present disclosure to more efficiently obtain the desired isomer of HFO-1132 than in conventional production methods.

### 2.1. Catalyst

Examples of catalysts include halides, oxides, and oxidized halides of transition metals, elements that belong to group 14 and group 15, and Mg, Al, etc. Specific examples of transition elements include Ti, V, Cr, Mn, Fe, Co, Ni, Cu, Zn, Zr, Nb, Ta, and W. Specific examples of elements that belong to group 14 include Sn and Pb. Specific examples of elements that belong to group 15 include Sb and Bi.

Examples of halides of these elements include fluorides and chlorides.

Of these, examples of preferable catalysts include SbCl₅, SbCl₃, SbF₅, TaCl₅, SnCl₄, NbCl₅, FeCl₃, CrCl₃, CrF₃, TiCl₄, MoCl₅, Cr₂O₃, CrO₂, CrO₃, CoCl₂, NiCl₂, MgF₂, AlOF (fluorinated aluminum oxide), and CrOF (fluorinated chromium oxide).

These catalysts may be used singly, or in a combination of two or more. They can be supported on a carrier. The carrier is not limited, and examples include porous alumina silicate typified by zeolite, aluminum oxide, silicon oxide, activated carbon, titanium oxide, zirconium oxide, zinc oxide, and aluminum fluoride. These may be used singly or in a combination of two or more, or a structural composite form thereof can be used. Specific examples of catalysts supported on a carrier include Cr_{y}O₃/Al₂O₃, Cr₂O₃/AlF₃, Cr₂O₃/C, CoCl₂/Cr₂O₃/Al₂O₃, NiCl₂/Cr₂O₃/Al₂O₃, CoCl₂/AlF₃, and NiCl₂/AlF₃.

As the catalyst, a chromium-containing catalyst is preferably used, and chromium oxide is particularly preferably used. Examples of chromium oxide include crystalline chromium oxide, amorphous chromium oxide, and the like, with the chromium oxide subjected to fluorination being particularly preferable.

### 2.2. Reactor

The reactor containing a catalyst is a rector in the form of a fixed bed or fluidized bed containing the catalyst described above. For example, catalysts in the form of pellets, powders, granules, or the like can be used.

### 2.3. Starting Material Gas

In the method according to Embodiment 1, when the starting material gas comprising HFC-143, HFO-1132(E) and/or HFO-1132(Z), and a diluent gas is supplied to the reactor, the content of the diluent gas in the starting material gas is 1 to 80 mol%. The content of HFC-143, and HFO-1132(E) and/or HFO-1132(Z) in the starting material gas can be freely set.

Since the content of the diluent gas in the starting material gas is 1 to 80 mol%, the formation of by-products in step (x) can be suppressed. The details of the diluent gas are described below. For example, the use of HF gas as the diluent gas can suppress the formation (production as a by-product) of HFC-143a in step (x) compared with the case in which no diluent gas is used. It is difficult to separate HFC-143a from HFO-1132(E); thus, by using HF gas as the diluent gas to suppress the formation of such by-products, the reaction product can be more easily separated into a first stream comprising HFO-1132(E) as a main component and a second stream comprising HFO-1132(Z) as a main component in subsequent step (y).

When at least one member selected from the group consisting of HF gas (hydrogen fluoride gas), O₂ gas (oxygen gas), CO₂ gas (carbon dioxide gas), and N₂ gas (nitrogen gas) is used as the diluent gas, catalyst degradation can be efficiently suppressed, and the amount of HFO-1132(E) and/or HFO-1132(Z) produced can be maintained for a long period of time in step (x). For example, when the case of using O₂ gas as the diluent gas is compared with the case of using no diluent gas, the conversion of HFC-143 in step (x) starts to decrease immediately after the start of the reaction when no diluent gas is used; however, when O₂ gas is used as the diluent gas, good conversion is maintained for 100 hours or more.

The content of the diluent gas in the starting material gas is preferably 4 to 70 mol%, and more preferably 40 to 65 mol%. In particular, by setting the diluent gas content to 40 to 65 mol%, the catalyst life can be easily extended. Although not bound by theory, this is presumably because the formation of a polymer and/or tar is efficiently suppressed.

In the method according to Embodiment 1, HF gas, O₂ gas, CO₂ gas, N₂ gas, helium gas, argon gas, and the like, can be used as the diluent gas. In particular, in terms of cost, N₂ gas is preferred. In addition, HF gas, O₂ gas, and CO₂ gas can each be a preferred diluent gas from the viewpoints described below.

In the case in which HF gas is used as the diluent gas, when the reaction product is separated into a first stream comprising HFO-1132(E) as a main component and a second stream comprising HFO-1132(Z) as a main component in the subsequent step (y), the second stream comprising HFO-1132(Z) as a main component can be suitably recycled to step (x) as a stream containing a desired amount of HF without completely separating the HF contained in the second stream comprising HFO-1132(Z) as a main component. This reduces the equipment and operating costs of step (y) compared with the case in which HF gas is not used.

When the diluent gas contains O₂ gas, the molar concentration of O₂ based on the total molar concentration of HFO-1132(E) and/or HFO-1132(Z), and HFC-143 is not limited. By setting the molar concentration of O₂ to preferably 5 mol% or more, more preferably 10 mol% or more, the effect of extending the catalyst life can be further enhanced. The use of chlorine gas in place of O₂ gas can also enhance the effect of extending the catalyst life. That is, chlorine gas can be used as the diluent gas.

Further, when the diluent gas contains CO₂ gas, the molar concentration of CO₂ based on the total molar concentration of HFO-1132(E) and/or HFO-1132(Z), and HFC-143 is not limited. By setting the molar concentration of CO₂ to preferably 30 mol% or more, more preferably 50 mol% or more, the effect of reducing the flammability of the starting material gas, the gas containing the reaction product, and the gas in the system subjected to the separation step can be obtained. The concentration of CO₂ in the diluent gas can be freely adjusted to reduce the flammability of the gas in the process system and further make it non-flammable, and can also be appropriately determined from the viewpoint of the yield of the desired product in the separation step.

### 2.4. Reaction Conditions

In step (x), a dehydrofluorination reaction of HFC-143, and an isomerization reaction between HFO-1132(E) and HFO-1132(Z) are performed to obtain a composition comprising HFO-1132(E) and HFO-1132(Z). HFO-1132(E) and/or HFO-1132(Z) is obtained by the dehydrofluorination reaction of HFC-143. In step (x), the dehydrofluorination reaction and the isomerization reaction are performed in the same reactor or in one step. In step (x) of the present disclosure, both reactions are basically performed in the same reactor. However, when the actual use of the same reactor is difficult, for example, when the size of the reactor is too large or the reactor is too long for engineering reasons, an embodiment in which multiple reactors controlled under the same reaction conditions are arranged in parallel instead of using the same reactor (one reactor), and starting material gases having the same composition are supplied to the individual reactors can be adopted. This embodiment is referred to as "one step" (the same applies to Embodiment 2 described later).

The dehydrofluorination reaction and the isomerization reaction may be performed either continuously or batch-wise. In terms of increasing production efficiency, the dehydrofluorination reaction and the isomerization reaction are preferably performed continuously. Both reactions can be performed in either a liquid phase or a gas phase, and the reactions are preferably performed in a gas phase. The following pressure and contact time conditions are for the gas phase reactions.

The reaction temperature in both reactions in step (x) is not limited, and is preferably within the range of 200°C to 500°C, and more preferably within the range of 300°C to 450°C.

The pressure in the reactor is not limited, and can be suitably set. Because a high pressure promotes the formation of a polymer such as tar, a suitable pressure can be set. The pressure is generally within the range of about ordinal pressure to 0.2 MPaG, and preferably within the range of about ordinal pressure to 0.1 MPaG. The pressure in the present disclosure refers to gauge pressure, unless otherwise specified ("G" is appended after the unit) .

The reaction time can be suitably set, and is not limited. An increase in contact time can increase the conversion; however, the catalyst amount is increased, which requires large equipment, and is thus inefficient. Accordingly, it is necessary to select a suitable contact time. The contact time represented by the ratio of the amount of the catalyst contained W (g) to the flow rate Fo (flow rate at 0°C and 1 atmosphere: cc/sec) of the starting material gas flowing to the reaction system, W/Fo, is generally preferably about 0.01 to 80 g·sec/cc, more preferably about 0.5 to 50 g·sec/cc, and even more preferably about 1 to 15 g·sec/cc. By setting the contact time within the above ranges, the formation of by-products can be easily suppressed.

### 2.5. Step (y)

The method according to Embodiment 1 may comprise step (y) of separating the reaction product obtained in step (x) into a first stream comprising HFO-1132(E) as a main component and a second stream comprising HFO-1132(Z) as a main component. For example, specifically, the gas at the reactor outlet produced by the isomerization reaction in step (x) is cooled to be liquefied and then distilled to be separated into a first stream comprising HFO-1132(E) as a main component and a second stream comprising HFO-1132(Z) as a main component. The first stream comprising HFO-1132(E) as a main component comprises HFO-1132(E) in an amount of 30 mol% or more, preferably 50 mol% or more, of the total organic matter in the first stream. The second stream comprising HFO-1132(Z) as a main component comprises HFO-1132(Z) in an amount of 30 mol% or more, preferably 50 mol% or more, of the total organic matter in the second stream.

The first stream may comprise, for example, HFC-143a, which is produced by the transition reaction of HFC-143 used for obtaining a composition comprising HFO-1132(E) and/or HFO-1132(Z), in addition to HFO-1132(E), which is the desired product. In this case, HFO-1132(E) can be further separated by means such as additional distillation.

### 2.6. Step (z)

The method according to Embodiment 1 may comprise step (z) of recycling the first stream or the second stream obtained in step (y) to step (x) to subject the first stream or the second stream to the isomerization reaction.

To collect a composition with a higher content of either HFO-1132(E) or HFO-1132(Z), it is preferred that the method according to Embodiment 1 further comprises collecting a stream (the first or second stream obtained in step (y)) that is different from the stream recycled in step (z). For example, the first stream may be recycled in step (z), and the step of collecting the second stream may be performed.

### 3. Embodiment 2: Embodiment in which W/Fo Ratio is Specific W/Fo Ratio

The method for producing difluoroethylene according to Embodiment 2 is a method for producing a composition comprising trans-1,2-difluoroethylene (HFO-1132(E)) and/or cis-1,2-difluoroethylene (HFO-1132(Z)), the method comprising
(x) supplying a starting material gas comprising 1,1,2-trifluoroethane (HFC-143), and HFO-1132(E) and/or HFO-1132(Z) to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein a contact time represented by the ratio of the amount of the catalyst contained W (g) to the flow rate of the starting material gas Fo (flow rate at 0°C and 1 atmosphere: cc/sec), W/Fo, is 0.1 to 10 g·sec/cc.

In step (x) in the method of Embodiment 2, a starting material gas comprising HFC-143, and HFO-1132(E) and/or HFO-1132(Z) is supplied to a reactor containing a catalyst to perform a dehydrofluorination reaction of HFC-143 and an isomerization reaction between HFO-1132(E) and HFO-1132(Z), thereby obtaining a composition comprising HFO-1132(E) and HFO-1132(Z). In the method of Embodiment 2, since the contact time represented by the ratio of the amount of the catalyst contained W (g) to the flow rate of the starting material gas Fo (flow rate at 0°C and 1 atmosphere: cc/sec), W/Fo, is 0.1 to 10 g·sec/cc, the formation of by-products can be suppressed. In particular, the formation of CO, CO₂, HFC-143a, etc. is suppressed, and when the reaction product is separated into a first stream comprising HFO-1132(E) as a main component and a second stream comprising HFO-1132(Z) as a main component in the subsequent step (y), the amount of HFO-1132 lost along with CO, CO₂, HFC-143a, etc. can be reduced. This allows the production method of the present disclosure to more efficiently obtain the desired isomer of HFO-1132 than in conventional production methods.

### 3.1. Catalyst

Examples of catalysts include halides, oxides, and oxidized halides of transition metals, elements that belong to group 14 and group 15, and Mg, Al, etc. Specific examples of transition elements include Ti, V, Cr, Mn, Fe, Co, Ni, Cu, Zn, Zr, Nb, Ta, and W. Specific examples of elements that belong to group 14 include Sn and Pb. Specific examples of elements that belong to group 15 include Sb and Bi.

Examples of halides of these elements include fluorides and chlorides.

Of these, examples of preferable catalysts include SbCl₅, SbCl₃, SbF₅, TaCl₅, SnCl₄, NbCl₅, FeCl₃, CrCl₃, CrF₃, TiCl₄, MoCl₅, Cr₂O₃, CrO₂, CrO₃, CoCl₂, NiCl₂, MgF₂, AlOF (fluorinated aluminum oxide), and CrOF (fluorinated chromium oxide).

These catalysts may be used singly, or in a combination of two or more. They can be supported on a carrier. The carrier is not limited, and examples include porous alumina silicate typified by zeolite, aluminum oxide, silicon oxide, activated carbon, titanium oxide, zirconium oxide, zinc oxide, and aluminum fluoride. These may be used singly or in a combination of two or more, or a structural composite form thereof can be used. Specific examples of catalysts supported on a carrier include Cr₂O₃/Al₂O₃, Cr₂O₃/AlF₃, Cr₂O₃/C, CoCl₂/Cr₂O₃/Al₂O₃, NiCl₂/Cr₂O₃/Al₂O₃, CoCl₂/AlF₃, and NiCl₂/AlF₃.

As the catalyst, a chromium-containing catalyst is preferably used, and chromium oxide is particularly preferably used. Examples of chromium oxide include crystalline chromium oxide, amorphous chromium oxide, and the like, with the chromium oxide subjected to fluorination being particularly preferable.

### 3.2. Reactor

The reactor containing a catalyst is a rector in the form of a fixed bed or fluidized bed containing the catalyst described above. For example, catalysts in the form of pellets, powders, granules, or the like can be used.

### 3.3. Starting Material Gas

In the method of Embodiment 2, a starting material gas comprising HFC-143, and HFO-1132(E) and/or HFO-1132(Z) is used. The content of HFC-143, and HFO-1132(E) and/or HFO-1132(Z) in the starting material gas can be freely set. The starting material gas may be diluted with a diluent gas.

As the diluent gas, HF gas, O₂ gas, CO₂ gas, N₂ gas, helium gas, argon gas, and the like can be used. In particular, in terms of cost, N₂ gas is preferred.

When the starting material gas contains a diluent gas, the formation of by-products can be suppressed, and catalyst degradation can be efficiently suppressed in step (x). Although not bound by theory, this is presumably because the formation of a polymer and/or tar is efficiently suppressed. The molar concentration of the diluent gas in the starting material gas is preferably 0.01 to 3.0 mol%, more preferably 0.1 to 2.0 mol%, and even more preferably 0.2 to 1.0 mol%, based on the total molar concentration of HFO-1132(E) and/or HFO-1132(Z), and HFC-143. When O₂ gas or chlorine gas is used as the diluent gas, the effect of extending the catalyst life can be further enhanced. That is, chlorine gas can also be used as the diluent gas.

When the diluent gas contains CO₂ gas, the molar concentration of CO₂ based on the total molar concentration of HFO-1132(E) and /or HFO-1132(Z), and HFC-143 is not limited. By setting the molar concentration of CO₂ to preferably 30 mol% or more, more preferably 50 mol% or more, the effect of reducing the flammability of the starting material gas, the gas containing the reaction product, and the gas in the system subjected to the separation step can be obtained. The concentration of CO₂ in the diluent gas can be freely adjusted to reduce the flammability of the gas in the process system and further to make it non-flammable, and can also be appropriately determined from the viewpoint of the yield of the desired product in the separation step.

### 3.4. Reaction Conditions

In step (x), a dehydrofluorination reaction of HFC-143 and an isomerization reaction between HFO-1132(E) and HFO-1132(Z) are performed to obtain a composition comprising HFO-1132(E) and HFO-1132(Z). HFO-1132(E) and/or HFO-1132(Z) is obtained by the dehydrofluorination reaction of HFC-143. In step (x), the dehydrofluorination reaction and the isomerization reaction are performed in the same reactor or in one step. Both reactions may be performed either continuously or batch-wise. In terms of increasing production efficiency, the reactions are preferably performed continuously. In Embodiment 2, the reactions are gas phase reactions.

In the method of Embodiment 2, when the starting material gas is supplied to the reactor, the contact time represented by the ratio of the amount of the catalyst contained W (g) to the flow rate of the starting material gas Fo (flow rate at 0°C and 1 atmosphere: cc/sec), W/Fo, is set to the range of 0.1 to 10 g·sec/cc. This can suppress the formation of by-products in step (x). In particular, the formation of CO, CO₂, HFC-143a, etc. is suppressed, and when the reaction product is separated into a first stream comprising HFO-1132(E) as a main component and a second stream comprising HFO-1132(Z) as a main component in the subsequent step (y), the amount of HFO-1132 lost along with CO, CO₂, HFC-143a, etc. can be reduced. This allows the production method of the present disclosure to more efficiently obtain the desired isomer of HFO-1132 than in conventional production methods. The contact time represented by W/Fo is within the range of 0.1 to 10 g·sec/cc, and preferably within the range of 1 to 5 g·sec/cc. By setting the contact time to the above ranges, the formation of by-products can be more easily suppressed.

The reaction temperature in both reactions in step (x) is not limited, and is preferably within the range of 200°C to 500°C, and more preferably within the range of 300°C to 450°C.

The pressure in the reactor is not limited, and can be suitably set. Because a high pressure promotes the formation of a polymer such as tar, a suitable pressure can be set. The pressure is generally within the range of about ordinal pressure to 0.2 MPaG, and preferably within the range of about ordinal pressure to 0.1 MPaG.

### 3.5. Step (y)

The method of Embodiment 2 may comprise step (y) of separating the reaction product obtained in step (x) into a first stream comprising HFO-1132(E) as a main component and a second stream comprising HFO-1132(Z) as a main component. For example, specifically, the gas at the reactor outlet produced by the isomerization reaction in step (x) is cooled to be liquefied and then distilled to be separated into a first stream comprising HFO-1132(E) as a main component and a second stream comprising HFO-1132(Z) as a main component. The first stream comprising HFO-1132(E) as a main component comprises HFO-1132(E) in an amount of 30 mol% or more, preferably 50 mol% or more, of the total organic matter in the first stream. The second stream comprising HFO-1132(Z) as a main component comprises HFO-1132(Z) in an amount of 30 mol% or more, preferably 50 mol% or more, of the total organic matter in the second stream.

The first stream may comprise, for example, HFC-143a, which is produced by the transition reaction of HFC-143 used for obtaining a composition comprising HFO-1132(E) and/or HFO-1132(Z), in addition to HFO-1132(E), which is the desired product. In this case, HFO-1132(E) can be further separated by means such as additional distillation.

### 3.6. Step (z)

The method of Embodiment 2 may comprise step (z) of recycling the first stream or the second stream obtained in step (y) to step (x) to subject the first stream or the second stream to the isomerization reaction.

To collect a composition with a higher content of either HFO-1132(E) or HFO-1132(Z), it is preferred that the method according to Embodiment 2 further comprises collecting a stream (the first or second stream obtained in step (y)) that is different from the stream recycled in step (z). For example, the first stream may be recycled in step (z), and the step of collecting the second stream may be performed.

### Examples

The present disclosure will be explained with reference to the Examples below; however, the present disclosure is not limited to these Examples.

### Examples 1 to 12

A process comprising supplying a starting material gas comprising HFC-143 and HFO-1132(Z) to a reactor containing a catalyst to subject the starting material gas to a dehydrofluorination reaction and an isomerization reaction, thereby obtaining a composition comprising HFO-1132(E) and HFO-1132(Z) (step (x)), sending the composition to a separation step to separate the composition into a first stream comprising HFO-1132(E) as a main component and a second stream comprising HFO-1132(Z) as a main component (step (y)), and recycling the second stream to step (x) (step (z)) was established.

### (1) Dehydrofluorination Reaction and Isomerization Reaction (Step (x) )

Under the reaction conditions shown in Tables 1 and 2, each starting material gas (shown by the formulation at the inlet) was subjected to a dehydrofluorination reaction and a isomerization reaction to obtain compositions comprising HFO-1132(E) and HFO-1132(Z) (shown by the organic matter formulations at the outlet) (step (x)). The reactor had an outer diameter of 12.7 mm and a length of 700 mm. 10 g of a chromium oxide catalyst obtained by the following preparation procedure was placed in the reactor, and the above reactions were performed.

### Preparation Procedure for Chromium Oxide Catalyst

114 g of 10% aqueous ammonia was added to 765 g of a 5.7% aqueous chromium nitrate solution, and the resulting precipitate was filtered and washed, followed by drying in air at 120°C for 12 hours, thereby obtaining chromium hydroxide. The chromium hydroxide was formed into pellets with a diameter of 3.0 mm and a height of 3.0 mm. The pellets were calcined at 400°C for 2 hours in a nitrogen stream. As a result of the determination of the Cr content and elemental analysis, the obtained chromium oxide was identified as CrO_{2.0}. The chromium oxide in pellet form was placed in a Hastelloy C reaction tube, a gas obtained by diluting HF with N₂ to 20 volume% was used, and heating was performed while gradually increasing the temperature from 200 to 360°C. After the temperature reached 360°C, fluorination was performed with 100% HF for 220 hours to obtain a fluorinated chromium oxide catalyst.

The results of step (x) in each of Examples 1 to 12 are as follows and are also summarized in Tables 1 and 2.

### Example 1

The formulation at the inlet (mol%) was 30.0/60.0/10.0/0.0, in the order of HFC-143/HFO-1132(Z)/O₂/HF.

The reaction temperature was 350°C, and the contact time represented by W/Fo was 10 g·sec/cc.

The organic matter formulation at the outlet (mol%) was 65.0/13.0/12.0/4.0/3.1/0.8, in the order of HFO-1132(Z)/HFO-1132(E)/HFC-143/CO/CO₂/R143a.

### Example 2

The formulation at the inlet (mol%) was 28.7/57.4/9.6/4.3, in the order of HFC-143/HFO-1132(Z)/O₂/HF.

The reaction temperature was 350°C, and the contact time represented by W/Fo was 10 g·sec/cc.

The organic matter formulation at the outlet (mol%) was 64.0/12.2/15.0/2.9/2.9/0.9, in the order of HFO-1132(Z)/HFO-1132(E)/HFC-143/CO/CO₂/R143a.

### Example 3

The formulation at the inlet (mol%) was 15.8/31.6/5.3/47.4, in the order of HFC-143/HFO-1132(Z)/O₂/HF.

The reaction temperature was 350°C, and the contact time represented by W/Fo was 10 g·sec/cc.

The organic matter formulation at the outlet (mol%) was 60.0/11.1/23.0/2.2/2.0/1.1, in the order of HFO-1132(Z)/HFO-1132(E)/HFC-143/CO/CO₂/R143a.

### Example 4

The formulation at the inlet (mol%) was 10.7/21.4/3.6/64.3, in the order of HFC-143/HFO-1132(Z)/O₂/HF.

The reaction temperature was 350°C, and the contact time represented by W/Fo was 10 g·sec/cc.

The organic matter formulation at the outlet (mol%) was 58.0/9.5/26.0/1.6/1.8/1.1, in the order of HFO-1132(Z)/HFO-1132(E)/HFC-143/CO/CO₂/R143a.

### Example 5

The formulation at the inlet (mol%) was 10.7/21.4/3.6/64.3, in the order of HFC-143/HFO-1132(Z)/O₂/HF.

The reaction temperature was 350°C, and the contact time represented by W/Fo was 20 g·sec/cc.

The organic matter formulation at the outlet (mol%) was 57.0/9.7/24.0/2.7/2.9/1.8, in the order of HFO-1132(Z)/HFO-1132(E)/HFC-143/CO/CO₂/R143a.

### Example 6

The formulation at the inlet (mol%) was 15.0/80.0/5.0/0.0, in the order of HFC-143/HFO-1132(Z)/O₂/HF.

The reaction temperature was 370°C, and the contact time represented by W/Fo was 10 g·sec/cc.

The organic matter formulation at the outlet (mol%) was 72.0/15.0/4.0/2.9/3.1/1.2, in the order of HFO-1132(Z)/HFO-1132(E)/HFC-143/CO/CO₂/R143a.

### Example 7

The formulation at the inlet (mol%) was 15.0/80.0/5.0/0.0, in the order of HFC-143/HFO-1132(Z)/O₂/HF.

The reaction temperature was 370°C, and the contact time represented by W/Fo was 5 g·sec/cc.

The organic matter formulation at the outlet (mol%) was 75.0/11.0/7.0/2.0/2.2/0.8, in the order of HFO-1132(Z)/HFO-1132(E)/HFC-143/CO/CO₂/R143a.

### Example 8

The formulation at the inlet (mol%) was 15.0/80.0/5.0/0.0, in the order of HFC-143/HFO-1132(Z)/O₂/HF.

The reaction temperature was 370°C, and the contact time represented by W/Fo was 1.5 g·sec/cc.

The organic matter formulation at the outlet (mol%) was 80.0/8.0/9.0/1.1/0.8/0.4, in the order of HFO-1132 (Z)/HFO-1132(E)/HFC-143/CO/CO₂/R143a.

### Example 9

The formulation at the inlet (mol%) was 15.0/80.0/5.0/0.0, in the order of HFC-143/HFO-1132(Z)/O₂/HF.

The reaction temperature was 370°C, and the contact time represented by W/Fo was 0.5 g·sec/cc.

The organic matter formulation at the outlet (mol%) was 81.0/6.0/11.0/0.5/0.9/0.2, in the order of HFO-1132(Z)/HFO-1132(E)/HFC-143/CO/CO₂/R143a.

### Example 10

The formulation at the inlet (mol%) was 88.0/10.0/2.0/0.0, in the order of HFC-143/HFO-1132(Z)/O₂/HF.

The reaction temperature was 300°C, and the contact time represented by W/Fo was 3 g·sec/cc.

The organic matter formulation at the outlet (mol%) was 13.0/0.2/85.0/0.2/0.2/0.1, in the order of HFO-1132(Z)/HFO-1132(E)/HFC-143/CO/CO₂/R143a.

### Example 11

The formulation at the inlet (mol%) was 88.0/10.0/2.0/0.0, in the order of HFC-143/HFO-1132(Z)/O₂/HF.

The reaction temperature was 300°C, and the contact time represented by W/Fo was 7 g·sec/cc.

The organic matter formulation at the outlet (mol%) was 19.0/1.0/79.0/0.4/0.4/0.1, in the order of HFO-1132(Z)/HFO-1132(E)/HFC-143/CO/CO₂/R143a.

### Example 12

The formulation at the inlet (mol%) was 15.8/31.6/5.3/47.4, in the order of HFC-143/HFO-1132(Z)/O₂/N₂.

The reaction temperature was 350°C, and the contact time represented by W/Fo was 10 g·sec/cc.

The organic matter formulation at the outlet (mol%) was 59.0/11.6/20.0/2.9/2.8/0.9, in the order of HFO-1132(Z)/HFO-1132(E)/HFC-143/CO/CO₂/R143a.

**Table 1**

| | Formulation at inlet (mol%) | | | | Temperature | W/Fo | Organic matter formulation at outlet (mol%) | | | | | |
|---|---|---|---|---|---|---|---|---|---|---|---|---|
| Example | HFC-143 | Z-1132 | O₂ | HF | °C | g·sec/cc | Z-1132 | E-1132 | HFC-143 | CO | CO₂ | R143a |
| 1 | 30.0 | 60.0 | 10.0 | 0.0 | 350 | 10 | 65.0 | 13.0 | 12.0 | 4.0 | 3.1 | 0.8 |
| 2 | 28.7 | 57.4 | 9.6 | 4.3 | 350 | 10 | 64.0 | 12.2 | 15.0 | 2.9 | 2.9 | 0.9 |
| 3 | 15.8 | 31.6 | 5.3 | 47.4 | 350 | 10 | 60.0 | 11.1 | 23.0 | 2.2 | 2.0 | 1.1 |
| 4 | 10.7 | 21.4 | 3.6 | 64.3 | 350 | 10 | 58.0 | 9.5 | 26.0 | 1.6 | 1.8 | 1.1 |
| 5 | 10.7 | 21.4 | 3.6 | 64.3 | 350 | 20 | 57.0 | 9.7 | 24.0 | 2.7 | 2.9 | 1.8 |
| 6 | 15.0 | 80.0 | 5.0 | 0.0 | 370 | 10 | 72.0 | 15.0 | 4.0 | 2.9 | 3.1 | 1.2 |
| 7 | 15.0 | 80.0 | 5.0 | 0.0 | 370 | 5 | 75.0 | 11.0 | 7.0 | 2.0 | 2.2 | 0.8 |
| 8 | 15.0 | 80.0 | 5.0 | 0.0 | 370 | 1.5 | 80.0 | 8.0 | 9.0 | 1.1 | 0.8 | 0.4 |
| 9 | 15.0 | 80.0 | 5.0 | 0.0 | 370 | 0.5 | 81.0 | 6.0 | 11.0 | 0.5 | 0.9 | 0.2 |
| 10 | 88.0 | 10.0 | 2.0 | 0.0 | 300 | 3 | 13.0 | 0.2 | 85.0 | 0.2 | 0.2 | 0.1 |
| 11 | 88.0 | 10.0 | 2.0 | 0.0 | 300 | 7 | 19.0 | 1.0 | 79.0 | 0.4 | 0.4 | 0.1 |

**Table 2**

| | Formulation at inlet (mol%) | | | | Temperature | W/Fo | Organic matter formulation at outlet (mol%) | | | | | |
|---|---|---|---|---|---|---|---|---|---|---|---|---|
| Example | HFC143 | Z-1132 | O₂ | N₂ | °C | g·sec/cc | Z-1132 | E-1132 | HFC-143 | CO | CO₂ | R143a |
| 12 | 15.8 | 31.6 | 5.3 | 47.4 | 350 | 10 | 59.0 | 11.6 | 20.0 | 2.9 | 2.8 | 0.9 |

The isomerization reaction was performed using the fluorinated chromium oxide catalyst to obtain reaction products in which the concentration of either of the isomers was higher than that in the supplied starting material compositions comprising HFO-1132(E) and/or HFO-1132(Z). It was found that by using O₂ gas as a diluent gas in Examples 1 to 12, catalyst deterioration was suppressed, and good conversion was maintained, even after 100 hours of reaction. A comparison of Example 3, which uses HF gas as a diluent gas, with Example 12, which uses N₂ gas as a diluent gas, shows that the formation (production as by-products) of CO, CO₂, etc. was more suppressed in Example 3, which uses HF gas. Further, the results show that compared with Example 1, which does not use HF gas, the formation (production as by-products) of CO, CO₂, etc. was more suppressed in Example 2, which uses HF gas even in a small amount. The results also show that even when the contact time is long (not satisfying the requirement of Embodiment 2), as in Example 5, the formation (production as a by-product) of CO can be significantly suppressed as long as the amount of the diluent gas satisfies the requirement of Embodiment 1. In all of the Examples, since the formation of by-products was suppressed in step (x), each reaction product was easily separated into a first stream comprising HFO-1132(E) as a main component and a second stream comprising HFO-1132(Z) as a main component by distillation in the subsequent step (y).

### (2) Reaction Process

Based on the above results, the production process shown schematically in Fig. 1 was established. The flow rate (kg/hr) of each component in each stream shown below is also summarized in Table 3. The flow rate of each component in each stream was as follows, in the order of HFO-1132(E)/HFO-1132(Z)/HFC-143/HFC-143a/total of CO and CO₂/HF.
F11: 0/0/8.8/0/0/0
S11: 5.6/70.9/72/0.63/0.48/41.7
S12: 5.6/0/0/0.63/0.48/0.03
S13: 0/70.9/72/0/0/41.67
S14: 0/70.9/72/0/0/39.3
S15: 0/0/0/0/0/2.4

The reaction conditions were as follows: the reaction temperature: 350°C; the reaction pressure: 0.1 MPaG; W/Fo = 6 g·sec/cc. The desired product was HFO-1132(E). The HF gas in the streams can be removed by using a deoxidation apparatus before the streams are sent to the next step. Deoxidation can be performed by any method, such as adsorption, distillation, or washing with water.

**Table 3**

| Stream | F11 | S11 | S12 | S13 | S14 | S15 |
|---|---|---|---|---|---|---|
| | Flow rate (kg/hr) | | | | | |
| HFO-1132(E) | 0 | 5.6 | 5.6 | 0 | 0 | 0 |
| HFO-1132(Z) | 0 | 70.9 | 0 | 70.9 | 70.9 | 0 |
| HFC-143 | 8.8 | 72 | 0 | 72 | 72 | 0 |
| HFC-143a | 0 | 0.63 | 0.63 | 0 | 0 | 0 |
| CO+CO₂ | 0 | 0.48 | 0.48 | 0 | 0 | 0 |
| HF | 0 | 41.7 | 0.03 | 41.67 | 39.3 | 2.4 |

### Description of the Reference Symbols

A. Reactor for dehydrofluorination reaction and isomerization reaction
B. Distillation column
C. Distillation column

## Claims

1. A method for producing a composition comprising trans-1,2-difluoroethylene (HFO-1132(E)) and/or cis-1,2-difluoroethylene (HFO-1132(Z)), the method comprising:
(x) supplying a starting material gas comprising 1,1,2-trifluoroethane (HFC-143), HFO-1132(E) and/or HFO-1132(Z), and a diluent gas to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein the content of the diluent gas in the starting material gas is 1 to 80 mol%.

2. A method for producing a composition comprising trans-1,2-difluoroethylene (HFO-1132(E)) and/or cis-1,2-difluoroethylene (HFO-1132(Z)), the method comprising:
(x) supplying a starting material gas comprising 1,1,2-trifluoroethane (HFC-143), and HFO-1132(E) and/or HFO-1132(Z) to a reactor containing a catalyst to perform a dehydrofluorination reaction of HFC-143 and an isomerization reaction between HFO-1132(E) and HFO-1132(Z), thereby obtaining a reaction product comprising HFO-1132(E) and HFO-1132(Z), wherein a contact time represented by the ratio of the amount of the catalyst contained W (g) to the flow rate of the starting material gas Fo (flow rate at 0°C and 1 atmosphere: cc/sec), W/Fo, is 0.1 to 10 g·sec/cc.

3. The production method according to claim 1, wherein the diluent gas is at least one member selected from the group consisting of HF gas, O₂ gas, CO₂ gas, and N₂ gas.

4. The production method according to claim 1 or 2, further comprising:
(y) separating the reaction product obtained in step (x) into a first stream comprising HFO-1132(E) as a main component and a second stream comprising HFO-1132(Z) as a main component.

5. The production method according to claim 4, further comprising:
(z) recycling the first stream or the second stream obtained in step (y) to step (x) to subject the first stream or the second stream to the isomerization reaction.
